(19) **Europäisches Patentamt / European Patent Office / Office européen des brevets**

(11) **EP 4 262 523 B1**

(12) # EUROPEAN PATENT SPECIFICATION

(45) Date of publication and mention
of the grant of the patent:
**18.03.2026  Bulletin 2026/12**

(21) Application number: **21824595.9**

(22) Date of filing: **14.12.2021**

(51) International Patent Classification (IPC):
$A61B\ 5/00^{(2006.01)}$    $A61N\ 1/04^{(2006.01)}$
$A61N\ 1/36^{(2006.01)}$    $A61N\ 7/00^{(2006.01)}$
$A61N\ 5/00^{(2006.01)}$    $A61N\ 2/00^{(2006.01)}$
$A61B\ 5/055^{(2006.01)}$    $A61B\ 5/242^{(2021.01)}$
$A61B\ 5/377^{(2021.01)}$    $A61B\ 6/00^{(2024.01)}$
$A61B\ 8/06^{(2006.01)}$    $G16H\ 30/40^{(2018.01)}$

(52) Cooperative Patent Classification (CPC):
A61N 1/0456; A61B 5/0042; A61B 5/055;
A61B 5/4064; A61B 5/489; A61B 6/037;
A61B 6/501; A61B 6/506; A61B 6/507;
A61B 6/5217; A61B 8/06; A61B 8/0808;
A61B 8/0816; A61B 8/085; A61B 8/5223;    (Cont.)

(86) International application number:
**PCT/EP2021/085573**

(87) International publication number:
**WO 2022/128970 (23.06.2022 Gazette 2022/25)**

(54) **METHOD AND SYSTEM FOR CALIBRATION OF BRAIN HEMODYNAMICS**

VERFAHREN UND SYSTEM ZUR KALIBRIERUNG DER GEHIRNHÄMODYNAMIK

PROCÉDÉ ET SYSTÈME D'ÉTALONNAGE D'HÉMODYNAMIQUE CÉRÉBRALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **15.12.2020  EP 20214003**

(43) Date of publication of application:
**25.10.2023   Bulletin 2023/43**

(73) Proprietor: **Koninklijke Philips N.V.
5656 AG Eindhoven (NL)**

(72) Inventors:
• **HUIJBERS, Willem**
**5656 AG Eindhoven (NL)**
• **VAN DOOREN, Marieke**
**5656 AG Eindhoven (NL)**
• **VAN EE, Raymond**
**5656 AG Eindhoven (NL)**
• **LAMERICHS, Rudolf Mathias Johannes Nicolaas**
**5656 AG Eindhoven (NL)**

• **LEUFKENS, Timmy Robertus Maria**
**5656 AG Eindhoven (NL)**
• **MATTERS, Marco**
**5656 AG Eindhoven (NL)**
• **WESTERINK, Joanne Henriëtte Desirée Monique**
**5656 AG Eindhoven (NL)**
• **DENISSEN, Adrianus Johannes Maria**
**5656 AG Eindhoven (NL)**

(74) Representative: **Philips Intellectual Property &
Standards
High Tech Campus 52
5656 AG Eindhoven (NL)**

(56) References cited:
**US-A1- 2009 156 955      US-A1- 2014 058 247
US-A1- 2017 340 260**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 262 523 B1

**(Cont. next page)**

- YUAN YI ET AL: "Cortical hemodynamic responses induced by low-intensity transcranial ultrasound stimulation of mouse cortex", NEUROIMAGE, ELSEVIER, AMSTERDAM, NL, vol. 211, 1 February 2020 (2020-02-01), XP086095601, ISSN: 1053-8119, [retrieved on 20200201], DOI: 10.1016/J.NEUROIMAGE.2020.116597
- SUH M ET AL: "Blood volume and hemoglobin oxygenation response following electrical stimulation of human cortex", NEUROIMAGE, ELSEVIER, AMSTERDAM, NL, vol. 31, no. 1, 15 May 2006 (2006-05-15), pages 66 - 75, XP024906484, ISSN: 1053-8119, [retrieved on 20060515], DOI: 10.1016/J.NEUROIMAGE.2005.11.030

(52) Cooperative Patent Classification (CPC): (Cont.)
**A61N 1/36014; A61N 1/36025; A61N 1/36034; A61N 2/006; G16H 20/30; G16H 30/40; G16H 50/20;** A61B 6/5235; A61B 6/54; A61B 8/5207; A61N 2007/0026; A61N 2007/0078; A61N 2007/0095; G16H 20/10

**Description**

FIELD OF THE INVENTION

The present invention is set out in the appended claims and generally relates to calibration of brain hemodynamics. The present disclosure describes a system for creating a hemodynamic brain atlas for calibration of brain hemodynamics, a device for calibration of brain hemodynamics, a neuromonitoring system, a method for creating a hemodynamic brain atlas, a computer-implemented method for calibration of brain hemodynamics, and a computer program element.

BACKGROUND OF THE INVENTION

Functional magnetic resonance imaging (fMRI) is limited by the use of a canonical hemodynamic response function (HRF) across the brain. The use of one single canonical HRF, which is the same across the entire brain and across individuals, may be a limitation. The limitation of canonical HRF can be mitigated partially, by estimation of HRF parameters in response to events (HRF calibration). However, this calibration can only be done in brain regions that have a tight coupling between events and evoked neuronal activity, i.e. the visual cortex.

US 2017/340260 AI describes a system for determining neurovascular reactivity to brain stimulation. US 2014/058247 A1 describes an information obtaining method of obtaining brain activity information from a living body to which a visual stimulus is given. Suh M et al.: "Blood volume and haemoglobin oxygenation response following electrical stimulation of human cortex", NeuroImage, Elsevier, Amsterdam, NL, vol. 31, no. 1, 15 May 2006, pages 66-75, reports the first measurements of deoxygenated hemoglobinin the human at a high spatial and temporal resolution.

SUMMARY

There may be a need to improve accuracy of brain hemodynamics calibration.

The object of the present invention is solved by the subject-matter of the independent claims, wherein further embodiments are incorporated in the dependent claims. It should be noted that the following described aspects apply also for the system for creating a hemodynamic brain atlas for calibration of brain hemodynamics, the device for calibration of brain hemodynamics, the neuromonitoring system, the method for creating a hemodynamic brain atlas, the computer-implemented method for calibration of brain hemodynamics, and the computer program element.

According to a first aspect , there is provided a system for creating a hemodynamic brain atlas for calibration of brain hemodynamics. The system comprises a non-invasive transcranial neurostimulation device, a non-invasive neuromonitoring device, and a computing device. The non-invasive transcranial neurostimulation device is configured to induce neuronal activity to evoke a hemodynamic response in a target region of interest (ROI) of a brain of a human subject. The non-invasive transcranial neurostimulation device comprises transcranial functional ultrasound stimulation (tFUS). The non-invasive neuromonitoring device is configured to monitor the evoked hemodynamic response in the target ROI. The computing device configured to determine a set of parameters representing a hemodynamic response function, HRF, of the evoked hemodynamic response in the target ROI, and to associate the set of parameters of the HRF with the target ROI to form the hemodynamic brain atlas.

In other words, it is proposed to use tFUS as the transcranial neurostimulation, to evoke neural activity and to subsequently estimate the HRF across various other brain regions. Neurostimulation has the advantage to be able to target any specified brain region, but also to be able to target multiple different brain regions. This may enable more accurate calibration of fMRI, not only in individuals, but also for specific brain regions.

TFUS is a non-invasive technique to evoke neuronal activity using low intensity ultrasound. TFUS can safely and effectively modulate neuronal activity in animals and humans. Neurostimulation using tFUS requires far less energy as compared to high-focused ultrasound (HIFU), used for ablation. TFUS is not limited to superficial layers of the cortex, but can target deep brain structures as well and can be highly focal, which was previously not possible in a non-invasive manner. Conventional techniques, such as transcranial direct current stimulation (tDCS) and transcranial magnetic stimulation (TMS), can only induce neuronal activity in regions close to skull, at the cortical surface, and have a large size (e.g. on the order of centimeters) of the modulatory area. In addition, the effectiveness of tFUS in cortical and deep brain structure may be improving with advent of tFUS systems that employ multiple ultrasound transducers, which can focus extra energy in localized deep brain structures. Therefore, with the tFUS, it is possible to determine the HRF across the entire brain to create a hemodynamic brain atlas. This will enable calibration of the HRF, not only in sensory-regions (e.g. sensory-motor, visual or auditory cortex), but across the entire brain.

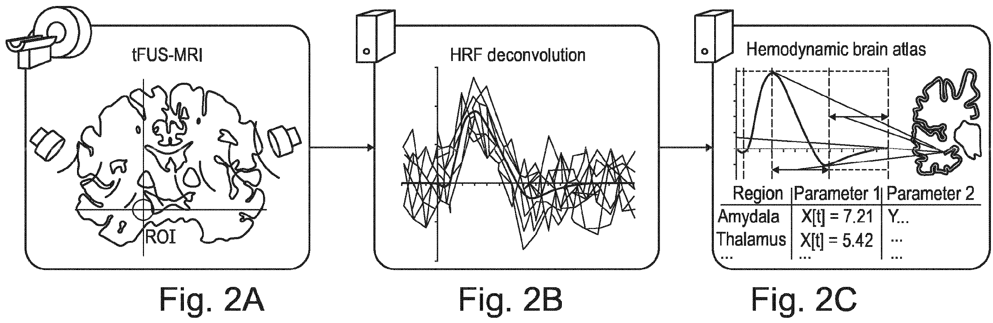

It is also proposed to develop a hemodynamic brain atlas that comprises a plurality of brain regions. Each brain region is associated with a respective hemodynamic response function (HRF) represented by a set of parameters. Thus, the hemodynamic brain atlas comprises a plurality of regionally calibrated HRFs. An exemplary hemodynamic brain atlas is illustrated in Fig. 2C. This atlas may improve the accuracy of fMRI measurement and could be extended by other methods of neurostimulation and neuromonitoring.

The target ROI may be anatomically, functionally, or clinically defined. The target ROI may be anatomically defined as the motor cortex, visual cortex, amygdala, a volume at an atlas coordinate, or a specific part of a brain region in the cortex (e.g. cortical layer IV of MT in the left hemisphere), sub-cortical region (e.g. substantia nigra, thalamus, hippocampus), or a region in the cerebellum. The target ROI can be functionally defined using and localizer task in the fMRI (motor, language, etc.), using functional connectivity, a functional atlas or using a PET (positron emission tomography) tracer. The target ROI may be clinically, for example as the foci of epileptic seizures or a stroke.

The set of parameters representing HRF may include one or more of peak amplitude, trough amplitude, time to peak, time to fall, rise slope, fall slope, FWHM (full width at half maximum, or area under the curve), etc.

According to the present description, the non-invasive transcranial neurostimulation device is configured to evoke a plurality of hemodynamic responses in a plurality of target ROIs, each hemodynamic response being evoked in a respective target ROI. The computing device is configured to determine, for each evoked hemodynamic response, a respective set of parameters representing the corresponding HRF and to associate each set of parameters with the corresponding target ROI to form the hemodynamic brain atlas.

For example, the non-invasive transcranial neurostimulation device may target multiple different brain regions consecutively or at the same time by multiple transducers.

According to an embodiment, the non-invasive neuromonitoring device is configured to monitor a further evoked hemodynamic response in one or more brain regions that have sufficiently strong excitatory connectivity with the target ROI. The computing device is configured to determine a set of parameters representing an HRF of the further evoked hemodynamic response in the one or more brain regions, and to associate the set of parameters with the one or more brain regions to form the hemodynamic brain atlas.

Accordingly, the HRF is not only estimated in the stimulated ROI, but also in other regions that might show a consistent BOLD response after stimulation.

For example, tFUS might also evoke a neuronal, and ensure BOLD response, in a distal brain region with strong excitatory connectivity.

According to an embodiment, the system further comprises a sensory stimulation device configured to apply at least one sensory stimulus over a human subject to induce neuronal activity to evoke a hemodynamic response in the target ROI. The computing device is configured to perform a comparison between the hemodynamic response evoked by the non-invasive transcranial neurostimulation device and the hemodynamic response evoked by the sensory stimulation device to correct a potential neurostimulation-induced confound on the HRF.

In other words, this system may also correct for potential neurostimulation-induced confounds on the HRF, for example via a comparison between tFUS-evoked HRFs and sensory/motor evoked HRF's. For one single distinct brain region, the deconvolution of the neurostimulation-evoked HRF may be compared to deconvolution of a sensory-evoked HRF.

The sensory stimulus may include one or more of visual stimulus, auditory stimulus, tactile stimulus, heat stimulus, etc.

According to an embodiment, the non-invasive transcranial neurostimulation device is configured to evoke a sequence of hemodynamic responses in the target ROI. The non-invasive neuromonitoring device is configured to monitor the sequence of evoked hemodynamic responses in the target ROI. The computing device is configured to perform a comparison among the sequence of evoked hemodynamic responses to correct a potential neurostimulation-induced confound on the HRF.

In an example, the non-invasive neuromonitoring device may be an MRI. The MRI may use specific fMRI sequences to better separate the influence of changes in cerebral blood flow and BOLD, such as vascular space occupancy (VASO) depended BOLD fMRI, or a FMR sequence that allows better estimation of the influence of temperature changes and BOLD.

According to an embodiment, the target ROI is selected from a set of calibration brain regions. An HRF in other brain regions of the brain is derivable from an HRF in the set of calibration brain regions.

An optimal set of calibration regions may be defined by exploring the regularities across brain regions. Although the HRF differs across brain regions, there are regularities (for example between hemispheres). Once a sufficient number of patients have been mapped into a hemodynamic brain atlas, these regularities can be exploited, and an optimal set of calibration regions can be defined.

Using a small set of individual calibration regions, for example one in each lobe, the method may infer a whole-brain hemodynamics.

According to an embodiment, the non-invasive transcranial neurostimulation device further comprises one or more of: transcranial electrical stimulation (tDCS/tACS), and transcranial magnetic stimulation (TMS).

In other words, other non-invasive brain stimulation methods, such as transcranial magnetic stimulation (TMS) and transcranial direct current stimulation (tDCS), may be used together with the tFUS to evoke neuronal activity.

According to an embodiment, the non-invasive neuromonitoring device comprises one or more of: a device for neuromonitoring of brain hemodynamics, and a device for measuring electrical signals produced by neurons to measure brain activity.

The hemodynamic neuroimaging device may include e.g. MRI, low intensity focused ultrasound imaging (LIFU), and near-infrared spectroscopy (NIRS).

Other methods of non-invasive neuromonitoring, e.g. electroencephalography (EEG), magnetoencephalography (MEG), Optically-Pumped Magnetometers (OPM), or motor-evoked responses, may be used to estimate the amount of neuronal activity induced by the neurostimulation.

According to a second aspect, a device for calibration of brain hemodynamics is provided. The device comprises an input unit, a processing unit, and an output unit. The input unit is configured to receive (i) a hemodynamic response in a brain region of interest (ROI) of a human subject acquired by a non-invasive neuromonitoring device and (ii) a hemodynamic brain atlas obtained according to the first aspect. The hemodynamic brain atlas comprises a plurality of brain regions, each brain region being associated with a respective hemodynamic response function, HRF, represented by a set of parameters. The processing unit is to calibrate the acquired hemodynamic response with the hemodynamic brain atlas. The output unit is configured to output the calibrated hemodynamic response.

In other words, a system is proposed for deploying the hemodynamic brain atlas, using the newly and correctly calibrated HRF, e.g. on an MRI system.

This hemodynamic brain atlas may include an average from a group of healthy adults, from a specified patient group, and/or from a single patient for whom many brain regions have been calibrated. This external atlas might also include HRF parameters that have been derived from different individuals for various brain regions.

According to an embodiment, the hemodynamic brain atlas is derivable from one or more of: previous HRF measurements of the human subject, previous HRF measurements of a group of healthy adults, optionally stratified by age, sex or other factors, previous HRF measurements of a specified patient group, and previous HRF measurements of a single patient for whom may brain regions have been calibrated.

According to an embodiment, the target ROI is different from the plurality of brain regions of the hemodynamic brain atlas.

The calibrated HRF may be applied to other brain regions than those that showed a response during calibration. For example, a hemodynamic brain atlas with a set of calibrated ROIs from the left hemisphere may be used to estimate brain activity in the contra-lateral hemisphere.

According to a third aspect , there is provided a neuromonitoring system. The neuromonitoring system comprises a non-invasive neuromonitoring device for acquiring a hemodynamic response in a region of interest (ROI) of a brain of a human subject, and a device according to the second aspect and any associated example for calibrating the acquired hemodynamic response.

According to a fourth aspect, there is provided a method for creating a hemodynamic brain atlas for calibration of brain hemodynamics, as claimed in claim 12.

According to a fifth aspect , there is provided a computer-implemented method for calibration of brain hemodynamics, as claimed in claim 13.

According to another aspect, there is provided a computer program element for controlling a system according to the first aspect and any associated example or a device according to the second aspect and any associated example, which when being executed by a processor is configured to carry out the method according to the third or fourth aspect.

It should be appreciated that all combinations of the foregoing concepts and additional concepts discussed in greater detail below (provided such concepts are not mutually inconsistent) are contemplated as being part of the subject matter disclosed herein. In particular, all combinations of claimed subject matter appearing at the end of this disclosure are contemplated as being part of the subject matter disclosed herein.

These and other aspects of the invention will be apparent from and elucidated with reference to the embodiment(s) described hereinafter. The present invention is set out in the appended claims.

BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.

Fig. 1 schematically shows an example of a system for creating a hemodynamic brain atlas for calibration of brain hemodynamics.

Figs. 2A-2C schematically shows an operation example of using the tFUS-MRI system for creating a hemodynamic brain atlas.

Fig. 3 schematically shows a further example of a system for creating a hemodynamic brain atlas for calibration of brain hemodynamics.

Fig. 4 schematically illustrates a device for calibration of brain hemodynamics.

Fig. 5 schematically shows an example of a neuromonitoring system.

Fig. 6 shows a flowchart of a method for creating a hemodynamic brain atlas for calibration of brain hemodynamics.

Fig. 7 shows a flowchart of a method for calibration of brain hemodynamics.

DETAILED DESCRIPTION OF EMBODIMENTS

Functional Magnetic Resonance Imaging (fMRI) is a non-invasive tool used to measure neuronal activity in response to a stimulus, task or under resting-state conditions. Unfortunately, fMRI does not directly measure the activity of neurons, i.e. action potentials of single cell neurons, but relies on the coupling between neuronal metabolism and blood flow. Functional MRI sequences typically employ the blood oxygen level-dependent (BOLD) contrast to estimate brain activity. Hence, fMRI depends on the response of blood vessels in the brain, and is therefore limited, and temporally filtered, by the hemodynamics of the brain's vasculature.

Event-related fMRI studies have identified a canonical hemodynamic response function (HRF) of the brain. An event, e.g. the presentation of short visual stimulus will evoke almost instantaneous neuronal activity in the visual cortex. In contrast to neuronal activity, the HRF is delayed. In the visual cortex, the stimulus evokes a delayed increase in BOLD signal, due to the dilation of the blood vessels and a large influx of oxygenated blood. This large increase in signal is the main source of fMRI. The HRF typically peaks at approximately 6 seconds after visual stimulation. After about 15-20 seconds, the HRF slowly returns to baseline. The HRF may be used to estimate brain activity in task fMRI, by convolution with the stimuli or task events. Furthermore, the HRF may be used to deconvolve fMRI time series and estimate neuronal events and task-evoked changes in brain connectivity. In sum, the HRF is a critical component of fMRI, in order to link changes in BOLD signal to events and vice versa.

The use of one single canonical HRF is considered a limitation, as it is the same across the entire brain and across individuals. Further, it has been shown that the HRF function is also age-dependent.

The above-mentioned limitation, e.g. the use of one single canonical HRF across the entire brain and across individuals, may be mitigated partially by estimation of HRF parameters in response to certain events, i.e. HRF calibration. This calibration can, however, only be done in brain regions that have a tight coupling between these events and evoked neuronal activity. For example, the visual cortex shows a reliable response to visual stimuli. Using fMRI, and the presentation of visual stimuli, the HRF can be deconvolved in the visual cortex and personalized HRF parameters can be derived. With similar methods, the HRF can be estimated for auditory and sensory-motor regions. However, most brain regions do not reliably modulate neuronal activity in response to a stimulus. Consequently, the HRF cannot be calibrated for most of the brain.

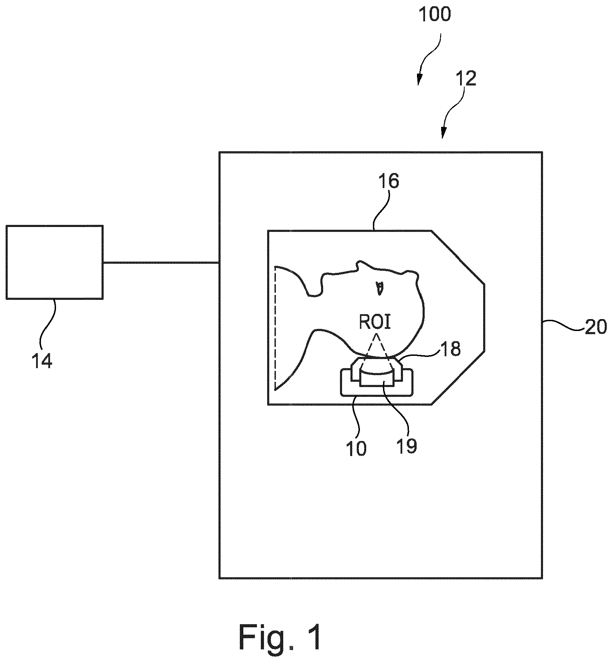

To address at least one of the above-mentioned limitations, Fig. 1 illustrates an example of a system 100 for creating a hemodynamic brain atlas for calibration of brain hemodynamics. The system 100 comprises a non-invasive transcranial neurostimulation device 10, a non-invasive neuromonitoring device 12, and a computing device 14. The target ROI may be e.g. the amygdala, a volume at an atlas coordinate, or a specific part of a brain region (e.g. cortical layer IV of MT in the left hemisphere).

The non-invasive transcranial neurostimulation device 10 is configured to induce neuronal activity to evoke a hemodynamic response in a target region of interest (ROI) of a brain of a human subject.

In the example shown in Fig. 1, the non-invasive transcranial neurostimulation device 10 is a transcranial focused ultrasound (tFUS) device. TFUS is a non-invasive technique to evoke neuronal activity using low intensity ultrasound. TFUS can safely and effectively modulate neuronal activity in animals and humans. Neurostimulation using tFUS requires far less energy as compared to high-focused ultrasound (HIFU), used for ablation. TFUS is not limited to superficial layers of the cortex, but can target deep brain structures as well and can be highly focal. In addition, the effectiveness of tFUS in cortical and deep brain structure is improving with advent of tFUS systems that employ multiple ultrasound transducers, which can focus extra energy in localized deep brain structures.

As shown in Fig. 1, the tFUS device is configured to transcranially deliver pulsed low-intensity FUS to the ROI, whereby similar ranges of frequencies may be employed to modulate human brain activity and to achieve adequate transcranial transmission through the skull. In the example shown in Fig. 1, the tFUS device is positioned inside an MR head coil 16 in an MRI system 20, with the illustration of the sonication path shown as dotted lines. The tFUS may be applied to the ROI through a coupling hydrogel 18, such as polyvinyl alcohol (PVA) hydrogel. The coupling hydrogel 18 may be around the contour of the skin to achieve tight acoustic coupling while maintaining the orientation of the sonication entry as perpendicular as possible to the scalp. In the example of Fig. 1, the tFUS device comprises a single FUS transducer 19 configured to evoke a hemodynamic response in a single target ROI. In other examples (not shown), the tFUS may be configured to evoke a plurality of hemodynamic responses in a plurality of target ROIs. Each hemodynamic response is evoked in a respective target ROI. This may be done by consecutively delivering pulsed low-intensity FUS to multiple ROIs with the single FUS transducer 19 or by simultaneously delivery multiple pulsed low-intensity FUS to multiple ROIs with multiple transducers (not shown). In other words, neurostimulation may target any specified brain region, but also (if desired) to be able to target multiple different brain regions.

Other non-invasive brain stimulation methods (not shown), such as transcranial magnetic stimulation (TMS) and transcranial direct current stimulation (tDCS), may be used together with the tFUS to evoke neuronal activity. However, the size of the modulatory area may be large (e.g. in the order of centimeters) while ability to reach specific regions in deep cortical/subcortical areas may be limited compared to tFUS.

The non-invasive neuromonitoring device 12 is configured to monitor the evoked hemodynamic response in the target ROI.

In some examples, functional neuroimaging modalities, such as functional magnetic resonance imaging (fMRI) and positron emission tomography (PET), may be used to characterize the spatial and temporal features of neural responses to FUS-mediated brain stimulation.

In the example shown in Fig. 1, the non-invasive neuromonitoring device 12 comprises an MRI system 20 that provides a high-resolution anatomical MRI that is usable for stereotactic guidance of tFUS. The MRI system 20 may also be used as the non-invasive neuromonitoring device 12. For example, fMRI may be used to measure neuronal activity in response to the stimulus. Functional MRI sequences typically employ the blood oxygen level-dependent (BOLD) contrast to estimate brain activity. Hence, fMRI depends on the response of blood vessels in the brain, and is therefore limited, and temporally filtered, by the hemodynamics of the brain's vasculature.

In some examples (not shown), the non-invasive neuromonitoring device 12 may perform hemodynamic imaging with low intensity focused ultrasound (LIFU) imaging or near-infrared spectroscopy. Both methods could also be combined with MRI for both neuromonitoring and/or stereotactic navigation. In the case of LIFU, the Doppler aspects of the ultrasound imaging may be used to obtain a measure of the (absolute) blood flow in real time. LIFU imaging may also be used to measure the blood flow in real time with high sensitivity, high spatiotemporal resolution (typically 100 $\mu$m and 10 ms) and large field of view (ranging from square cm to tens of square cm). LIFU is sometimes called functional ultrasound imaging. Thereby, LIFU could be used instead, or together, with fMRI measurements, to deconvolve the HRF and improve calibration for novel fMRI or LIFU measurements. Furthermore, using LIFU, it is possible to measure the blood flow in the blood vessel of interest before ($F_{min}$) and at the peak of the tFUS stimulus ($F_{max}$). Whilst LIFU imaging provides absolute values of the flow rate, this could be even improved upon by using anatomical imaging of the size of the blood vessel to calculate the total change in blood volume. For example, in the example of visual stimulus, it can be expected that the size of the blood vessel will change from initial area of blood vessel ($A_{min}$) and after 6 seconds reach its largest size (e.g. area of blood vessel $A_{max}$). Hence, if a second more dedicated scan (i.e. a higher resolution etc.) is taken after 6 seconds the change in (absolute) blood flow is calculated by

$$V_{max} = V_{min} \times (F_{max} \times A_{\max})/(F_{min} \times A_{min})$$

This is another way to calibrate the BOLD response using LIFU and fMRI measurement. In addition, any change resulting from thermal events (i.e. changes in blood viscosity) caused by the stimulus may also be identified by e.g. using near-infrared spectroscopy.

In some further examples (not shown), the non-invasive neuromonitoring device 12 may measure the brain's electrical currents to estimate the amount of neuronal activity induced by the non-invasive transcranial neurostimulation device 10. Exemplary non-invasive neuromonitoring methods may include, but are not limited to, electroencephalography (EEG), magnetoencephalography (MEG), Optically-Pumped Magnetometers (OPM), and motor-evoked responses. The additional estimates of neuronal activity may be used to infer the expected HRF. These independent estimates of neuronal activity can help further improve the calibration procedure and inform e.g. tFUS parameters, including the intensity, frequency or duration of tFUS.

The computing device 14 is configured to determine a set of parameters representing a hemodynamic response function (HRF) of the evoked hemodynamic response in the target ROI. The computing device 14 is further configured to associate the set of parameters of the HRF with the target ROI to form the hemodynamic brain atlas. The set of parameters representing HRF may include one or more of peak amplitude, trough amplitude, time to peak, time to fall, rise slope, fall slope, FWHM, etc.

When the hemodynamic brain atlas is acquired before and after medication, the influence of this medication on several regions or networks may be registered, for instance to investigate the effects of medication. This could be used to assess the influence of medication on brain activity, for example psychoactive medicinal drugs. This may enable personal medication to its full potential: each person has different susceptibility for brain medication. Side effects of brain medication may be high and can be predicted at forehand.

An operation example of using a tFUS-MRI system is shown in Figs. 2A-2C. A patient will be placed inside the MRI system 20. First, as shown in Fig. 2A, a high-resolution anatomical MRI is made for stereotactic guidance of tFUS and to register the various measurements. One or more tFUS devices will then be used to evoke a HRF in one or more target ROIs (e.g. the amygdala, a volume at an atlas coordinate, or a specific part of a brain region). During, and immediately following transcranial neurostimulation, the evoked hemodynamic response will be measured using fMRI. This can be compared with baseline measurements without stimulation or sham. The scanner will be optimized to monitor the ROI with a high temporal and spatial resolution in order to sample the shape of the hemodynamic response and filter potential confounders. From each measurement, an HRF can be deconvolved and the function parameters estimated. This procedure may be repeated several times to decrease noise and estimate variance in the HRF. An exemplary HRF deconvolution is shown in Fig. 2B. The HRF deconvolution results in individualized HRF parameters (e.g. peak amplitude, trough amplitude, time to peak, time to fall, rise slope, fall slope, and/or FWHM) for the stimulated brain ROI. Using the information of the anatomical MRI, the HRF parameters will be mapped onto a hemodynamic brain atlas. An exemplary hemodynamic brain atlas is shown in Fig. 2C, which is mapped onto a stereotactic space and stored in a digital file.

In the example of Figs. 2A-2C, there is a single brain region that is determined by the ROI and the spatial resolution of tFUS (and fMRI). The same calibration procedure can be repeated to calibrate the HRF for a series of other brain regions, resulting in a hemodynamic brain atlas.

The regionally calibrated HRFs based on hemodynamic brain atlas may be used during acquisition of novel fMRI measurements. The estimation of new fMRI activity works akin to current statistical parametric mapping studies, but instead of a canonical HRF, the regionally calibrated HRFs are convolved to estimate fMRI activity. Thus, a more accurate estimation of brain activity of the patient is obtained, which supports the formation of a correct conclusion or diagnosis.

Optionally, the target ROI(s) may be selected from a set of calibration brain regions. An HRF in other brain regions of the brain may be derivable from an HRF in the set of calibration brain regions. Although the HRF differs across brain regions, there are regularities (for example between hemispheres). For example, once a sufficient number of patients have been mapped into a hemodynamic brain atlas, these regularities may be exploited, and an optimal set of calibration regions may be defined. For example, following stimulation, the HRF in the entorhinal cortex might on average peak half a second later than the HRF in the hippocampus. Also, in one patient the HRF might have a 20% larger amplitude than another patient. The set of calibration brain regions may take these regularities between brain regions and individuals into account. By calibrating the HRF in a single (or optimal set) of brain regions, the HRF parameters will be inferred for un-calibrated brain region. In this way, the hemodynamic brain atlas will be extended using a transfer function. This means that using a small set of individual calibration regions, for example one in each lobe, the method will infer a whole-brain hemodynamics.

Optionally, the non-invasive neuromonitoring device 12 may be configured to monitor a further evoked hemodynamic response in one or more brain regions that have sufficiently strong excitatory connectivity with the target ROI. The computing device 14 may be configured to determine a set of parameters representing an HRF of the further evoked hemodynamic response in the one or more brain regions, and to associate the set of parameters with the one or more brain regions to form the hemodynamic brain atlas. In other words, the HRF is not only estimated in the stimulated ROI(s), but also in other regions that might show a consistent BOLD response after stimulation. This is because neuronal activity is known to propagate to brain regions with dense excitatory connections. In some instances, neurostimulation might therefore also evoke a neuronal, and ensure BOLD response, in a distal brain region with strong excitatory connectivity. Using the comparison with baseline measurements made without stimulation, the system could calibrate multiple brain regions using neurostimulation of only a single ROI or a small number of ROIs.

Optionally, the non-invasive transcranial neurostimulation device 10 may be configured to evoke a sequence of hemodynamic responses in the target ROI. The non-invasive neuromonitoring device 12 is configured to monitor the sequence of evoked hemodynamic responses in the target ROI. The computing device 14 is configured to perform a comparison among the sequence of evoked hemodynamic responses to correct a potential neurostimulation-induced confound on the HRF. For example, the non-invasive transcranial neurostimulation device may be an MRI. The MRI may use fMRI sequences to better separate the influence of changes in cerebral blood flow and BOLD, such as vascular space occupancy dependend fMRI, or a FMR sequence that allows better estimation of the influence of temperature changes and BOLD.

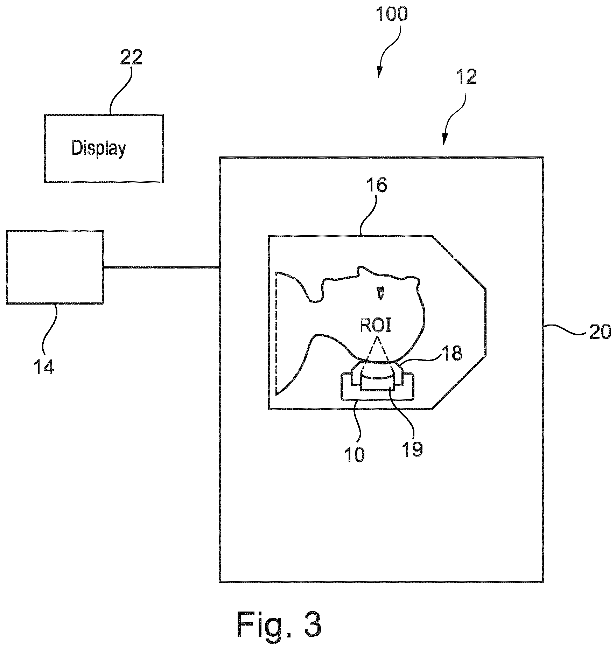

Fig. 3 illustrates a further example of a system 100 for creating a hemodynamic brain atlas for calibration of brain hemodynamics. In this example, the system 100 further comprises a sensory stimulation device 22 configured to apply at least one sensory stimulus over a human subject to induce neuronal activity to evoke a hemodynamic response in the target ROI.

In the example illustrated in Fig. 3, the sensory stimulation device 22 is a visual stimulation device. The visual stimulation device may be a screen, such as a computer monitor or a television, for displaying any combination of still images and video for predetermined durations on predetermined areas of the screen. The visual stimuli may be brought into the scanner using a mirror arrangement to allow the patient to view an (inverted) display in the mirror, by a projection of the visual stimuli onto the bore of the MRI, by providing the patient with MRI compatible head mounted displays etc.

In another example (not shown), the sensory stimulation device 22 may be an auditory stimulation device, such as a speaker connected to an audio source and outputting predetermined sounds at predetermined timings according to the control signal.

In a further example (not shown), the sensory stimulation device 22 may be a tactile stimulation device, such as a vibrating device that is touching (or very close to) the human subject and in response to the control signal, vibrates at predetermined vibration strength and frequency and at predetermined timings.

The computing device 14 may be configured to perform a comparison between the hemodynamic response evoked by the non-invasive transcranial neurostimulation device and the hemodynamic response evoked by the sensory stimulation device to correct a potential neurostimulation-induced confound on the HRF.

In other words, for one single distinct brain region the deconvolution of the neurostimulation-evoked HRF may be compared to a deconvolution of a sensory-evoked HRF. This direct comparison may enable the system to control for neurostimulation-induced confounds on the HRF. Slight tissue heating (within the safety margins) might result in a larger HRF response. The calibration procedure includes a comparison of sensory-evoked HRF in the same brain region, thereby creating an additional reference. For example, the visual cortex may be calibrated twice, using visual stimulation via a projector and using tFUS as shown in Fig. 3. The comparison between both types of stimulation will allow for estimation and correction of tFUS induced influence on the HRF.

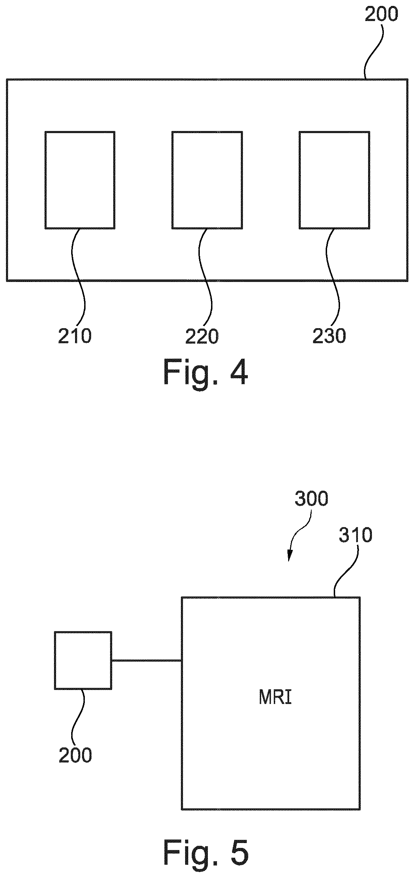

Fig. 4 schematically illustrates a device 200 for calibration of brain hemodynamics. The device 200 comprises an input unit 210, a processing unit 220, and an output unit 230.

The input unit 210 is configured to receive (i) a hemodynamic response in a region of interest (ROI) of a brain of a human subject acquired by a non-invasive neuromonitoring device and (ii) a hemodynamic brain atlas.

In some examples, the hemodynamic response may be obtained using functional neuroimaging modalities, such as fMRI and PET. In some examples, the hemodynamic response may be obtained using hemodynamic imaging with low intensity focused ultrasound imaging or near infrared spectroscopy.

The hemodynamic brain atlas comprises a plurality of brain regions. Each brain region is associated with a respective hemodynamic response function (HRF) represented by a set of parameters, such as peak amplitude, trough amplitude, time to peak, time to fall, rise slope, fall slope, FWHM, etc. An exemplary hemodynamic brain atlas is illustrated in Fig. 2B.

In some examples, the hemodynamic brain atlas may include parameters based on prior information from measurements made at earlier times for the same patient. For example, it is possible to combine several HRF measurements done on the same brain area of the same person over the course of a number of weeks or years. This may also be considered a diagnostic tool in itself: it might show how the shape and timing of the HRF in a certain brain area changes or deteriorates over time.

In some examples, the hemodynamic brain atlas may include parameters based on prior information from measurements made at earlier times in other patients. Such hemodynamic brain atlas may also be referred to as "external atlas". For example, the hemodynamic brain atlas may be derivable from previous HRF measurements of a group of healthy adults, optionally stratified by age, sex or other factors, previous HRF measurements of a specified patient group, and previous HRF measurements of a single patient for whom may brain regions have been calibrated. This external atlas might also include HRF parameters that have been derived from different individuals for various brain regions. For example, the visual cortex might have been estimated in one patient, but the amygdala in another patient, etc. Before application, the system or an operator, could use various patient characteristics (e.g. age, gender, diagnosis) to select the most appropriate hemodynamic brain atlas.

The input unit 210 is, in an example, implemented as an Ethernet interface, a USB (TM) interface, a wireless interface such as a WiFi (TM) or Bluetooth (TM) or any comparable data transfer interface enabling data transfer between input peripherals and the processing unit 220.

The processing unit 220 is configured to calibrate the acquired hemodynamic response with the hemodynamic response atlas. The calibrated HRF based on hemodynamic brain atlas is used during acquisition of novel fMRI measurements. For example, the estimation of fMRI activity works akin to current statistical parametric mapping studies, but instead of a canonical HRF, the regionally calibrated HRFs are convolved to estimate fMRI activity. Thus, a more accurate estimation of brain activity of the patient is obtained, which supports the formation of a correct conclusion or diagnosis.

In some examples, the target ROI(s) may be different from the plurality of brain regions of the hemodynamic brain atlas. In other words, the calibrated HRF in the hemodynamic brain atlas may be applied to other brain regions than those that showed a response during calibration. For example, a hemodynamic brain atlas with a set of calibrated ROIs from the left hemisphere may be used to estimate brain activity in the contra-lateral hemisphere. During novel fMRI acquisition, a larger set of regions may be used that include the brain regions in the contra-lateral hemisphere, regardless of whether these additional regions showed an evoked BOLD response following stimulation of the ROIs.

The processing unit 220 may refer to, be part of, or include an Application Specific Integrated Circuit (ASIC), an electronic circuit, a processor (shared, dedicated, or group) and/or memory (shared, dedicated, or group) that execute one or more software or firmware programs, a combinational logical circuit, and/or other suitable components that provide the described functionality. Furthermore, such processing unit 14 may be connected to volatile or non-volatile storage, display interfaces, communication interfaces and the like as known to a person skilled in the art.

The output unit 230 is configured to output the calibrated hemodynamic response.

Fig. 5 schematically shows an example of a neuromonitoring system 300. The neuromonitoring system 300 comprises a non-invasive neuromonitoring device 310 and a device 200 as described with respect to Fig. 4.

The non-invasive neuromonitoring device 310 is configured for acquiring a hemodynamic response in a region of interest (ROI) of a brain of a human subject.

In the example of Fig. 5, the non-invasive neuromonitoring device 310 is an MRI device. In other examples (not shown), the non-invasive neuromonitoring device 310 may perform functional neuroimaging with PET.

The device 200 is configured for calibrating the acquired hemodynamic response, e.g. by convolving regionally calibrated HRFs in the hemodynamic brain atlas to estimate fMRI activity.

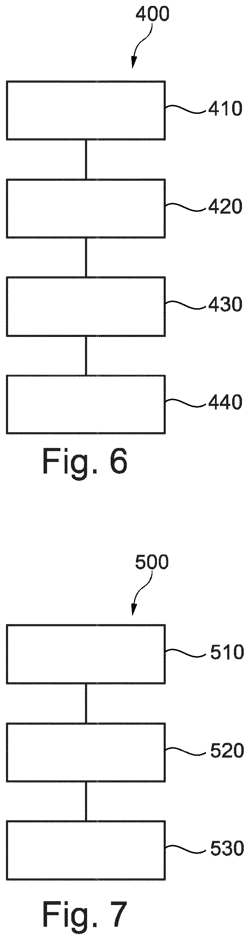

Fig. 6 illustrates a flowchart of a method 400 for creating a hemodynamic brain atlas for calibration of brain hemodynamics.

In step 410, a non-invasive transcranial neurostimulation device induces neuronal activity to evoke a hemodynamic response in a target region of interest (ROI) of a brain of a human subject.

The non-invasive transcranial neurostimulation device may include one or more of tFUS, tDCS/tACS, and TMS.

The target ROI may be anatomically, functionally or clinically defined. The ROI can be anatomically defined as the motor cortex, visual cortex, amygdala, a volume at an atlas coordinate, or a specific part of a brain region in the cortex (e.g. cortical layer IV of MT in the left hemisphere), sub-cortical region (e.g. substantia nigra, thalamus, hippocampus), or a region in the cerebellum. The ROI can be functionally defined using and localizer task in the fMRI (motor, language, etc.), using functional connectivity, a functional atlas or using a PET tracer. The ROI can be clinically, for example as the foci of epileptic seizures or a stroke.

If desired, the non-invasive transcranial neurostimulation device may target multiple different brain regions consecutively with a single transducer or at the same time by multiple transducers.

In step 420, a non-invasive neuromonitoring device monitors the evoked hemodynamic response in the target ROI.

The non-invasive neuromonitoring device may include one or more of fMRI, PET, Functional Ultrasound, LIFU, NIRS, EEG, MEG, and OPM.

In step 430, a computing device determines a set of parameters representing an HRF of the evoked hemodynamic response in the target ROI. The set of parameters may include one or more of peak amplitude, trough amplitude, time to peak, time to fall, rise slope, fall slope, FWHM, etc.

In step 440, the computing device associates the set of parameters of the HRF with the target ROI to form the hemodynamic brain atlas. The hemodynamic brain atlas thus comprises multiple regionally calibrated HRFs.

Fig. 7 shows a flowchart of a method 500 for calibration of brain hemodynamics.

In step 510, an input unit receives a hemodynamic response in a region of interest (ROI) of a brain of a human subject acquired by a non-invasive neuromonitoring device and (ii) a hemodynamic brain atlas. The hemodynamic brain atlas comprises a plurality of brain regions, each brain region being associated with a respective hemodynamic response function (HRF) represented by a set of parameters.

In some examples, the hemodynamic brain atlas may include parameters based on prior information from measurements made at earlier times for the same patient.

In some examples, the hemodynamic brain atlas may include parameters based on prior information from measurements in other patients, which is also referred to as external atlas. This external hemodynamic brain atlas could consist of an average from a group of healthy adults, from a specified patient group, or from a single patient for whom many brain regions have been calibrated.

In step 520, a processing unit calibrates the acquired hemodynamic response with the hemodynamic brain atlas.

In step 530, an output unit outputs the calibrated hemodynamic response.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary will refer to the inclusion of exactly one element of a number or list of elements.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In another exemplary embodiment, a computer program or a computer program element is provided that is characterized by being adapted to execute the method steps of the method according to one of the preceding embodiments, on an appropriate system.

The computer program element might therefore be stored on a computer unit, which might also be part of an embodiment.

This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment covers both, a computer program that right from the beginning uses the concepts according to the present disclosure and a computer program that by means of an up-date turns an existing program into a program that uses concepts according to the present disclosure.

Further on, the computer program element might be able to provide all necessary steps to fulfil the procedure of an exemplary embodiment of the method as described above.

According to a further exemplary embodiment, a computer readable medium, such as a CD-ROM, is presented wherein the computer readable medium has a computer program element stored on it, which computer program element is described by the preceding section.

A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment, a medium for making a computer program element available for downloading is provided, which computer program element is arranged to perform a method according to one of the previously described embodiments.

While several embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific embodiments described herein. It is, therefore, to be understood that the foregoing embodiments are presented by way of example only and that, within the scope of the appended claims and equivalents thereto, embodiments may be practiced otherwise than as specifically described . Embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

**Claims**

1. A system (100) for creating a hemodynamic brain atlas for calibration of brain hemodynamics, the system comprising:

- a non-invasive transcranial neurostimulation device (10) configured to induce neuronal activity to evoke a hemodynamic response in a target region of interest, ROI, of a brain of a human subject, wherein the non-invasive transcranial neurostimulation device comprises transcranial functional ultrasound stimulation, tFUS;
- a non-invasive neuromonitoring device (12) configured to monitor the evoked hemodynamic response in the target ROI; and
- a computing device (14) configured to determine a set of parameters representing a hemodynamic response function, HRF, of the evoked hemodynamic response in the target ROI, and to associate the set of parameters of the HRF with the target ROI to form the hemodynamic brain atlas,

wherein the non-invasive transcranial neurostimulation device is configured to evoke a plurality of hemodynamic responses in a plurality of target ROIs, each hemodynamic response being evoked in a respective target ROI; and

wherein the computing device is configured to determine, for each evoked hemodynamic response, a respective set of parameters representing the corresponding HRF and to associate each set of parameters with the corresponding target ROI to form the hemodynamic brain atlas usable for calibration of brain hemodynamics.

2. System according to claim 1,

wherein the non-invasive neuromonitoring device is configured to monitor a further evoked hemodynamic response in one or more brain regions that have sufficiently strong excitatory connectivity with the target ROI; and

wherein the computing device is configured to determine a set of parameters representing an HRF of the further evoked hemodynamic response in the one or more brain regions, and to associate the set of parameters with the one or more brain regions to form the hemodynamic brain atlas.

3. System according to claim 1 or 2, further comprising:

- a sensory stimulation device (22) configured to apply at least one sensory stimulus over a human subject to induce neuronal activity to evoke a hemodynamic response in the target ROI,

wherein the computing device is configured to perform a comparison between the hemodynamic response evoked by the non-invasive transcranial neurostimulation device and the hemodynamic response evoked by the sensory stimulation device to correct a potential neurostimulation-induced confound on the HRF.

4. System according to any one of the preceding claims,

wherein the non-invasive transcranial neurostimulation device is configured to evoke a sequence of hemodynamic responses in the target ROI;

wherein the non-invasive neuromonitoring device is configured to monitor the sequence of evoked hemodynamic responses in the target ROI; and

wherein the computing device is configured to perform a comparison among the sequence of evoked hemodynamic responses to correct a potential neurostimulation-induced confound on the HRF.

5. System according to any one of the preceding claims,

wherein the target ROI is selected from a set of calibration brain regions; and

wherein an HRF in other brain regions of the brain is derivable from an HRF in the set of calibration brain regions.

6. System according to any one of the preceding claims,
wherein the non-invasive transcranial neurostimulation device further comprises one or more of:

- transcranial electrical stimulation, tDCS/tACS; and
- transcranial magnetic stimulation, TMS.

7. System according to any one of the preceding claims,
wherein the non-invasive neuromonitoring device comprises one or more of:

- a device for neuromonitoring of brain hemodynamics; and
- a device for measuring electrical signals produced by neurons to measure brain activity.

8. A device (200) for calibration of brain hemodynamics, the device comprising:

- an input unit (210) configured to receive (i) a hemodynamic response in a region of interest, ROI, of a brain of a human subject acquired by a non-invasive neuromonitoring device and (ii) a hemodynamic brain atlas obtained according to any one of the preceding claims;

wherein the hemodynamic brain atlas comprises a plurality of brain regions, each brain region being associated with a respective hemodynamic response function, HRF, represented by a set of parameters;
- a processing unit (220) configured to calibrate the acquired hemodynamic response with the hemodynamic brain atlas; and
- an output unit (230) configured to output the calibrated hemodynamic response.

9. Device according to claim 8,
   wherein the hemodynamic brain atlas is derivable from one or more of:

   - previous HRF measurements of the human subject;
   - previous HRF measurements of a group of healthy adults;
   - previous HRF measurements of a specified patient group; and
   - previous HRF measurements of a single patient for whom brain regions may have been calibrated.

10. Device according to claim 8 or 9,
    wherein the ROI is different from the plurality of brain regions of the hemodynamic brain atlas.

11. A neuromonitoring system (300), comprising:

    - a non-invasive neuromonitoring device (310) for acquiring a hemodynamic response in a region of interest, ROI, of a brain of a human subject; and
    - a device according to any one of claims 8 to 10 for calibrating the acquired hemodynamic response.

12. A method (400) for creating a hemodynamic brain atlas for calibration of brain hemodynamics, the method comprising:

    - inducing (410), by a non-invasive transcranial neurostimulation device, neuronal activity to evoke a hemodynamic response in a target region of interest, ROI, of a brain of a human subject, wherein the non-invasive neuromonitoring device comprises transcranial functional ultrasound stimulation, tFUS;
    - monitoring (420), by a non-invasive neuromonitoring device, the evoked hemodynamic response in the target ROI;
    - determining (430), by a computing device, a set of parameters representing a hemodynamic response function, HRF, of the evoked hemodynamic response in the target ROI; and
    - associating (440), by the computing device, the set of parameters of the HRF with the target ROI to form the hemodynamic brain atlas,

      wherein the non-invasive transcranial neurostimulation device evokes a plurality of hemodynamic responses in a plurality of target ROIs, each hemodynamic response being evoked in a respective target ROI; and
      wherein the computing device determines, for each evoked hemodynamic response, a respective set of parameters representing the corresponding HRF and associates each set of parameters with the corresponding target ROI to form the hemodynamic brain atlas usable for calibration of brain hemodynamics.

13. A computer-implemented method (500) for calibration of brain hemodynamics, the method comprising:

    - receiving (510), by an input unit, (i) a hemodynamic response in a region of interest, ROI, of a brain of a human subject acquired by a non-invasive neuromonitoring device and (ii) a hemodynamic brain atlas obtained according to claim 12;
      wherein the hemodynamic brain atlas comprises one or more brain regions, each brain region being associated with a respective hemodynamic response function, HRF, represented by a set of parameters;
    - calibrating (520), by a processing unit, the acquired hemodynamic response with the hemodynamic brain atlas; and
    - outputting (530), by an output unit, the calibrated hemodynamic response.

14. A computer program element for controlling a system according to any one of claims 1 to 7 or a device according to any one of claims 8 to 10, which when being executed by a processor is configured to carry out the method according to claim 12 or 13.

**EP 4 262 523 B1**

**Patentansprüche**

1. System (100) zur Erstellung eines hämodynamischen Hirnatlas zur Kalibrierung der Hirn-Hämodynamik, wobei das System Folgendes umfasst:

   - eine nicht-invasive transkranielle Neurostimulationsvorrichtung (10), die konfiguriert ist, um neuronale Aktivität zu induzieren, um eine hämodynamische Reaktion in einer Zielregion von Interesse, ROI, eines Gehirns eines menschlichen Subjekts hervorzurufen, wobei die nicht-invasive transkranielle Neurostimulationsvorrichtung eine transkranielle funktionelle Ultraschallstimulation, tFUS, umfasst;
   - eine nicht-invasive Neuromonitoring-Vorrichtung (12), die konfiguriert ist, um die hervorgerufene hämodynamische Reaktion in der Ziel-ROI zu überwachen; und
   - eine Rechenvorrichtung (14), die konfiguriert ist, um einen Satz von Parametern zu bestimmen, die eine hämodynamische Reaktionsfunktion, HRF, der hervorgerufenen hämodynamischen Reaktion im Ziel-ROI darstellen, und den Satz von Parametern der HRF der Ziel-ROI zuzuordnen, um den hämodynamischen Hirnatlas zu bilden,

   wobei die nicht-invasive transkranielle Neurostimulationsvorrichtung konfiguriert ist, um eine Vielzahl hämodynamischer Reaktionen in einer Vielzahl von Ziel-ROIs hervorzurufen, wobei jede hämodynamische Reaktion in einer jeweiligen Ziel-ROI hervorgerufen wird; und
   wobei die Rechenvorrichtung konfiguriert ist, um für jede hervorgerufene hämodynamische Reaktion einen entsprechenden Satz von Parametern zu bestimmen, welche die entsprechende HRF darstellen, und um jeden Satz von Parametern der entsprechenden Ziel-ROI zuzuordnen, um den hämodynamischen Hirnatlas zu bilden, der zur Kalibrierung der Hirnhämodynamik verwendbar ist.

2. System nach Anspruch 1,

   wobei die nicht-invasive Neuromonitoring-Vorrichtung konfiguriert ist, um eine weitere hervorgerufene hämodynamische Reaktion in einer oder mehreren Hirnregionen zu überwachen, die eine ausreichend starke anregende Konnektivität mit der Ziel-ROI aufweisen; und
   wobei die Rechenvorrichtung konfiguriert ist, um einen Satz von Parametern zu bestimmen, die eine HRF der weiteren hervorgerufenen hämodynamischen Reaktion in einer oder mehreren Hirnregionen darstellen, und um den Satz von Parametern der einen oder mehreren Hirnregionen zuzuordnen, um den hämodynamischen Hirnatlas zu bilden.

3. System nach Anspruch 1 oder 2, weiter umfassend:

   - eine sensorische Stimulationsvorrichtung (22), die konfiguriert ist, um mindestens einen sensorischen Reiz auf ein menschliches Subjekt anzuwenden, um neuronale Aktivität zu induzieren, und eine hämodynamische Reaktion in der Ziel-ROI hervorzurufen,

   wobei die Rechenvorrichtung konfiguriert ist, um einen Vergleich zwischen der durch die nicht-invasive transkranielle Neurostimulationsvorrichtung hervorgerufenen hämodynamischen Reaktion und der durch die sensorische Stimulationsvorrichtung hervorgerufenen hämodynamischen Reaktion durchzuführen, um eine potenzielle, durch die Neurostimulation induzierte Störgröße in der HRF zu korrigieren.

4. System nach einem der vorstehenden Ansprüche,

   wobei die nicht-invasive transkranielle Neurostimulationsvorrichtung konfiguriert ist, um eine Abfolge hämodynamischer Reaktionen in der Ziel-ROI hervorzurufen;
   wobei die nicht-invasive Neuromonitoring-Vorrichtung konfiguriert ist, um die Abfolge hervorgerufener hämodynamischer Reaktionen in der Ziel-ROI zu überwachen; und
   wobei die Rechenvorrichtung konfiguriert ist, um einen Vergleich aus der Abfolge von hervorgerufenen hämodynamischen Reaktionen durchzuführen, um eine mögliche durch Neurostimulation induzierte Störgröße in der HRF zu korrigieren.

5. System nach einem der vorstehenden Ansprüche,
   wobei die Ziel-ROI aus einem Satz von Kalibrierungs-Hirnregionen ausgewählt wird; und wobei eine HRF in anderen Hirnregionen des Gehirns aus einer HRF in dem Satz an Kalibrierungs-Hirnregionen ableitbar ist.

**6.** System nach einem der vorstehenden Ansprüche,
wobei die nicht-invasive transkranielle Neurostimulationsvorrichtung weiter eines oder mehr umfasst von:

- transkranieller elektrischer Stimulation, tDCS/tACS; und
- transkranieller Magnetstimulation, TMS.

**7.** System nach einem der vorstehenden Ansprüche,
wobei die nicht-invasive Neuromonitoring-Vorrichtung eines oder mehr umfasst von:

- einer Vorrichtung zum Neuromonitoring von Hirnhämodynamik; und
- einer Vorrichtung zur Messung elektrischer Signale, die von Neuronen erzeugt werden, um die Hirnaktivität zu messen.

**8.** Vorrichtung (200) zur Kalibrierung von Hirnhämodynamik, wobei die Vorrichtung Folgendes umfasst:

- eine Eingabeeinheit (210), die konfiguriert ist, um (i) eine hämodynamische Reaktion in einer Region von Interesse, ROI, eines Gehirns eines menschlichen Subjekts, die mit einer nicht-invasiven Neuromonitoring-Vorrichtung erfasst wurde, und (ii) einen hämodynamischen Hirnatlas zu empfangen, der gemäß einem der vorhergehenden Ansprüche erhalten wurde;
wobei der hämodynamische Hirnatlas eine Vielzahl von Hirnregionen umfasst, wobei jede Hirnregion einer entsprechenden hämodynamischen Reaktionsfunktion, HRF, zugeordnet ist, die durch einen Satz von Parametern dargestellt wird;
- eine Verarbeitungseinheit (220), die konfiguriert ist, um die erfasste hämodynamische Reaktion mit dem hämodynamischen Hirnatlas zu kalibrieren; und
- eine Ausgabeeinheit (230), die konfiguriert ist, um die kalibrierte hämodynamische Reaktion auszugeben.

**9.** Vorrichtung nach Anspruch 8,
wobei der hämodynamische Hirnatlas von einem oder mehr ableitbar ist von:

- vorherigen HRF-Messungen des menschlichen Subjekts;
- vorherigen HRF-Messungen einer Gruppe gesunder Erwachsener;
- vorherigen HRF-Messungen einer bestimmten Patientengruppe; und
- vorherigen HRF-Messungen eines einzelnen Patienten, für den Hirnregionen hätten kalibriert werden können.

**10.** Vorrichtung nach Anspruch 8 oder 9,
wobei sich die ROI von der Vielzahl von Hirnregionen des hämodynamischen Hirnatlas unterscheidet.

**11.** Neuromonitoring-System (300), umfassend:

- eine nicht-invasive Neuromonitoring-Vorrichtung (310) zur Erfassung einer hämodynamischen Reaktion in einer Region von Interesse, ROI, eines Gehirns eines menschlichen Subjekts; und
- eine Vorrichtung nach einem der Ansprüche 8 bis 10 zur Kalibrierung der erfassten hämodynamischen Reaktion.

**12.** Verfahren (400) zur Erstellung eines hämodynamischen Hirnatlas zur Kalibrierung der Hirn-Hämodynamik, wobei das Verfahren Folgendes umfasst:

- Induzieren (410), durch eine nicht-invasive transkranielle Neurostimulationsvorrichtung, von neuronaler Aktivität, um eine hämodynamische Reaktion in einer Zielregion von Interesse, ROI, eines Gehirns eines menschlichen Subjekts hervorzurufen, wobei die Neuromonitoring-Vorrichtung eine transkranielle funktionelle Ultraschallstimulation, tFUS, umfasst;
- Überwachen (420), durch eine nicht-invasive Neuromonitoring-Vorrichtung, der hervorgerufenen hämodynamischen Reaktion in der Ziel-ROI;
- Bestimmen (430), durch eine Rechenvorrichtung, eines Satzes von Parametern, der eine hämodynamische Reaktionsfunktion, HRF, der hervorgerufenen hämodynamischen Reaktion in der Ziel-ROI darstellt; und
- Zuordnen (440), durch die Rechenvorrichtung, des Satzes von Parametern der HRF der Ziel-ROI, um den hämodynamischen Hirnatlas zu bilden, wobei die nicht-invasive transkranielle Neurostimulationsvorrichtung eine Vielzahl hämodynamischer Reaktionen in einer Vielzahl von Ziel-ROIs hervorruft, wobei jede hämodyna-

mische Reaktion in einem jeweiligen Ziel-ROI hervorgerufen wird; und

wobei die Rechenvorrichtung für jede hervorgerufene hämodynamische Reaktion einen entsprechenden Satz von Parametern bestimmt, welche die entsprechende HRF darstellen, und jeden Satz von Parametern der entsprechenden Ziel-ROI zuordnet, um den hämodynamischen Hirnatlas zu bilden, der zur Kalibrierung der Hirnhämodynamik verwendbar ist.

13. Computerimplementiertes Verfahren (500) zur Kalibrierung der Hirnhämodynamik, wobei das VerfahrenFolgendes umfasst:

- Empfangen (510), durch eine Eingabeeinheit, (i) einer hämodynamische Reaktion in einer Region von Interesse, ROI, eines Gehirns eines menschlichen Subjekts, die mit einer nicht-invasiven Neuromonitoring-Vorrichtung erfasst wurde, und (ii) eines hämodynamischen Hirnatlas, der gemäß Anspruch 12 erhalten wurde; wobei der hämodynamische Hirnatlas eine oder mehrere Hirnregionen umfasst, wobei jede Hirnregion einer entsprechenden hämodynamischen Reaktionsfunktion, HRF, zugeordnet ist, die durch einen Satz von Parametern dargestellt wird;
- Kalibrieren (520), durch eine Verarbeitungseinheit, der erfassten hämodynamischen Reaktion mit dem hämodynamischen Hirnatlas; und
- Ausgeben (530), durch eine Ausgabeeinheit, der kalibrierten hämodynamischen Reaktion.

14. Computerprogrammelement zur Steuerung eines Systems nach einem der Ansprüche 1 bis 7 oder einer Vorrichtung nach einem der Ansprüche 8 bis 10, das bei Ausführung durch einen Prozessor konfiguriert ist, um das Verfahren nach Anspruch 12 oder 13 durchzuführen.

## Revendications

1. Système (100) de création d'un atlas de l'hémodynamique cérébrale destiné à l'étalonnage de l'hémodynamique cérébrale, le système comprenant :

- un dispositif de neurostimulation transcrânienne non invasive (10) configuré pour induire une activité neuronale afin de provoquer une réponse hémodynamique dans une région d'intérêt, ROI, cible du cerveau d'un sujet humain, dans lequel le dispositif de neurostimulation transcrânienne non invasive comprend une stimulation ultrasonore fonctionnelle transcrânienne, tFUS ;
- un dispositif de neuromonitoring non invasif (12) configuré pour surveiller la réponse hémodynamique provoquée dans la ROI cible ; et
- un dispositif informatique (14) configuré pour déterminer un ensemble de paramètres représentant une fonction de réponse hémodynamique, HRF, de la réponse hémodynamique provoquée dans la ROI cible, et pour associer l'ensemble des paramètres de la HRF à la ROI cible afin de former l'atlas de l'hémodynamique cérébrale,

dans lequel le dispositif de neurostimulation transcrânienne non invasive est configuré pour provoquer une pluralité de réponses hémodynamiques dans une pluralité de ROI cibles, chaque réponse hémodynamique étant provoquée dans une ROI cible respective ; et

dans lequel le dispositif informatique est configuré pour déterminer, pour chaque réponse hémodynamique provoquée, un ensemble respectif de paramètres représentant la HRF correspondante et pour associer chaque ensemble de paramètres à la ROI cible correspondante afin de former l'atlas de l'hémodynamique cérébrale pouvant être utilisé pour l'étalonnage de l'hémodynamique cérébrale.

2. Système selon la revendication 1,

dans lequel le dispositif de neuromonitoring non invasif est configuré pour surveiller une réponse hémodynamique provoquée supplémentaire dans une ou plusieurs régions cérébrales présentant une connectivité excitatrice suffisamment forte avec la ROI cible ; et

dans lequel le dispositif informatique est configuré pour déterminer un ensemble de paramètres représentant une HRF de la réponse hémodynamique provoquée supplémentaire dans les une ou plusieurs régions cérébrales, et pour associer l'ensemble de paramètres aux une ou plusieurs régions cérébrales afin de former l'atlas de l'hémodynamique cérébrale.

3. Système selon la revendication 1 ou 2, comprenant en outre :

- un dispositif de stimulation sensorielle (22) configuré pour appliquer au moins un stimulus sensoriel sur un sujet humain afin d'induire une activité neuronale pour provoquer une réponse hémodynamique dans la ROI cible,

dans lequel le dispositif informatique est configuré pour effectuer une comparaison entre la réponse hémodynamique provoquée par le dispositif de neurostimulation transcrânienne non invasive et la réponse hémodynamique provoquée par le dispositif de stimulation sensorielle afin de corriger un éventuel biais induit par la neurostimulation sur la HRF.

4. Système selon l'une quelconque des revendications précédentes,

dans lequel le dispositif de neurostimulation transcrânienne non invasive est configuré pour provoquer une séquence de réponses hémodynamiques dans la ROI cible ;
dans lequel le dispositif de neuromonitoring non invasif est configuré pour surveiller la séquence des réponses hémodynamiques provoquées dans la ROI cible ; et
dans lequel le dispositif informatique est configuré pour effectuer une comparaison entre la séquence des réponses hémodynamiques provoquées afin de corriger un éventuel biais induit par la neurostimulation sur la HRF.

5. Système selon l'une quelconque des revendications précédentes,
dans lequel la ROI cible est choisie parmi un ensemble de régions cérébrales d'étalonnage ; et dans lequel une HRF dans d'autres régions cérébrales du cerveau peut être dérivée d'une HRF dans l'ensemble des régions cérébrales d'étalonnage.

6. Système selon l'une quelconque des revendications précédentes,
dans lequel le dispositif de neurostimulation transcrânienne non invasive comprend en outre un ou plusieurs éléments parmi :

- la stimulation électrique transcrânienne, tDCS/tACS ; et
- la stimulation magnétique transcrânienne, TMS.

7. Système selon l'une quelconque des revendications précédentes,
dans lequel le dispositif de neuromonitoring non invasif comprend un ou plusieurs éléments parmi :

- un dispositif de neuromonitoring de l'hémodynamique cérébrale ; et
- un dispositif permettant de mesurer les signaux électriques produits par les neurones afin de mesurer l'activité cérébrale.

8. Dispositif (200) d'étalonnage de l'hémodynamique cérébrale, le dispositif comprenant :

- une unité d'entrée (210) configurée pour recevoir (i) une réponse hémodynamique dans une région d'intérêt, ROI, du cerveau d'un sujet humain acquise par un dispositif de neuromonitoring non invasif et (ii) un atlas de l'hémodynamique cérébrale obtenu selon l'une quelconque des revendications précédentes ;
dans lequel l'atlas de l'hémodynamique cérébrale comprend une pluralité de régions cérébrales, chaque région cérébrale étant associée à une fonction de réponse hémodynamique, HRF, respective représentée par un ensemble de paramètres ;
- une unité de traitement (220) configurée pour étalonner la réponse hémodynamique acquise avec l'atlas de l'hémodynamique cérébrale ; et
- une unité de sortie (230) configurée pour produire la réponse hémodynamique étalonnée.

9. Dispositif selon la revendication 8,
dans lequel l'atlas de l'hémodynamique cérébrale peut être dérivé d'un ou plusieurs éléments parmi :

- les mesures de HRF précédentes du sujet humain ;
- les mesures de HRF précédentes d'un groupe d'adultes en bonne santé ;
- les mesures de HRF précédentes d'un groupe de patients déterminé ; et
- les mesures de HRF précédentes d'un seul patient pour lequel les régions cérébrales ont pu être étalonnées.

**10.** Dispositif selon la revendication 8 ou 9,
dans lequel la ROI est différente de la pluralité de régions cérébrales de l'atlas de l'hémodynamique cérébrale.

**11.** Système de neuromonitoring (300), comprenant :

- un dispositif de neuromonitoring non invasif (310) permettant d'acquérir une réponse hémodynamique dans une région d'intérêt, ROI, du cerveau d'un sujet humain ; et
- un dispositif selon l'une quelconque des revendications 8 à 10 pour étalonner la réponse hémodynamique acquise.

**12.** Procédé (400) de création d'un atlas de l'hémodynamique cérébrale pour l'étalonnage de l'hémodynamique cérébrale, le procédé comprenant :

- l'induction (410), par un dispositif de neurostimulation transcrânienne non invasive, d'une activité neuronale pour provoquer une réponse hémodynamique dans une région d'intérêt, ROI, cible du cerveau d'un sujet humain, dans lequel le dispositif de neuromonitoring non invasif comprend une stimulation ultrasonore fonctionnelle transcrânienne, tFUS ;
- la surveillance (420), par un dispositif de neuromonitoring non invasif, de la réponse hémodynamique provoquée dans la ROI cible ;
- la détermination (430), par un dispositif informatique, d'un ensemble de paramètres représentant une fonction de réponse hémodynamique, HRF, de la réponse hémodynamique provoquée dans la ROI cible ; et
- l'association (440), par le dispositif informatique, de l'ensemble de paramètres de la HRF à la ROI cible pour former l'atlas de l'hémodynamique cérébrale, dans lequel le dispositif de neurostimulation transcrânienne non invasive provoque une pluralité de réponses hémodynamiques dans une pluralité de ROI cibles, chaque réponse hémodynamique étant provoquée dans une ROI cible respective ; et

dans lequel le dispositif informatique détermine, pour chaque réponse hémodynamique provoquée, un ensemble respectif de paramètres représentant la HRF correspondante et associe chaque ensemble de paramètres à la ROI cible correspondante afin de former l'atlas de l'hémodynamique cérébrale pouvant être utilisé pour l'étalonnage de l'hémodynamique cérébrale.

**13.** Procédé mis en œuvre par ordinateur (500) pour l'étalonnage de l'hémodynamique cérébrale, le procédé comprenant :

- la réception (510), par une unité d'entrée, (i) d'une réponse hémodynamique dans une région d'intérêt, ROI, du cerveau d'un sujet humain acquise par un dispositif de neuromonitoring non invasif et (ii) d'un atlas de l'hémodynamique cérébrale obtenu selon la revendication 12 ;
dans lequel l'atlas de l'hémodynamique cérébrale comprend une ou plusieurs régions cérébrales, chaque région cérébrale étant associée à une fonction de réponse hémodynamique, HRF, respective représentée par un ensemble de paramètres ;
- l'étalonnage (520), par une unité de traitement, de la réponse hémodynamique acquise avec l'atlas de l'hémodynamique cérébrale ; et
- la production (530), par une unité de sortie, de la réponse hémodynamique étalonnée.

**14.** Élément de programme informatique destiné à commander un système selon l'une quelconque des revendications 1 à 7 ou un dispositif selon l'une quelconque des revendications 8 à 10, qui, lorsqu'il est exécuté par un processeur, est configuré pour mettre en œuvre le procédé selon la revendication 12 ou 13.

Fig. 1

tFUS-MRI

ROI

**Fig. 2A**

HRF deconvolution

**Fig. 2B**

Hemodynamic brain atlas

| Region | Parameter 1 | Parameter 2 |
|---|---|---|
| Amydala | X[t] = 7.21 | Y... |
| Thalamus | X[t] = 5.42 | ... |
| ... | ... | ... |

**Fig. 2C**

Fig. 3

200

210    220    230

Fig. 4

300

310

200

MRI

Fig. 5

400

410

420

430

440

Fig. 6

500

510

520

530

Fig. 7

**EP 4 262 523 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2017340260 A

- US 2014058247 A1

### Non-patent literature cited in the description

- Blood volume and haemoglobin oxygenation response following electrical stimulation of human cortex. **SUH M et al.** NeuroImage. Elsevier, 15 May 2006, vol. 31, 66-75